Europäisches Patentamt

(19) European Patent Office

Office européen des brevets

(11) EP 0 594 760 B1

(12) **FASCICULE DE BREVET EUROPEEN**

(45) Date de publication et mention
de la délivrance du brevet:
**06.05.1998 Bulletin 1998/19**

(21) Numéro de dépôt: **92915962.2**

(22) Date de dépôt: **10.07.1992**

(51) Int. Cl.$^6$: **A61K 9/08**, A61K 47/32

(86) Numéro de dépôt international:
**PCT/FR92/00664**

(87) Numéro de publication internationale:
**WO 93/00887 (21.01.1993 Gazette 1993/03)**

(54) **COMPOSITIONS OPHTALMIQUES A BASE D'ACIDE POLYACRYLIQUE**

OPHTHALMISCHE ZUSAMMENSETZUNGEN BERUHEND AUF POLYAKRYLSAEURE

POLYACRYLIC ACID BASED OPHTHALMIC COMPOSITIONS

(84) Etats contractants désignés:
**AT BE CH DE DK ES FR GB GR IT LI LU MC NL
SE**

(30) Priorité: **10.07.1991 FR 9108625**

(43) Date de publication de la demande:
**04.05.1994 Bulletin 1994/18**

(73) Titulaire:
**LABORATOIRE EUROPHTA S.A.
98000 Monaco (MC)**

(72) Inventeur: **SCHWADROHN, Gérard
F-06100 Nice (FR)**

(74) Mandataire:
**Hubert, Philippe et al
Cabinet Beau de Loménie
158, rue de l'Université
75340 Paris Cédex 07 (FR)**

(56) Documents cités:
**EP-A- 0 312 208         WO-A-90/13284
GB-A- 2 007 091**

Remarques:
Le dossier contient des informations techniques
présentées postérieurement au dépôt de la
demande et ne figurant pas dans le présent
fascicule.

## Description

La présente invention concerne de nouvelles compositions pharmaceutiques destinées à l'usage ophtalmique ainsi qu'à leurs procédés de préparation.

Elle a plus particulièrement pour objet de nouvelles compositions destinées à l'instillation oculaire et présentant des propriétés d'adhérence et/ou de rémanence très améliorées.

On sait, en effet, que lors de l'instillation de préparations ophtalmiques, le réflexe palpébral entraîne l'évacuation rapide d'une quantité non négligeable de cette préparation par le canal lacrymal et par là, dans les fosses nasales.

Pour remédier à une telle situation, on peut avoir recours à des solutions plus concentrées en pensant que le petit volume qui demeurera au contact du globe oculaire ou du sac lacrymal atteindra l'organe cible et aura une meilleure efficacité. Cependant, ces solutions ophtalmiques ont des inconvénients encore plus grands que les solutions diluées. Les solutions hypertoniques sont douloureuses. Les solutions trop concentrées laissent des dépôts sur le globe oculaire, gênent la vision et surtout sont mal tolérées localement. Les solutions concentrées ont, en outre, l'inconvénient d'entraîner plus fréquemment des effets secondaires indésirables liés à un effet systémique.

On a déjà eu également recours à des solutés hypervisqueux que l'on applique sur la paupière ou sur le globe oculaire. De telles préparations sont décrites dans le brevet français 2.407.714 (TOKO YAKUHIN KOGYO) ou dans le brevet européen 0.166.061. En fait, ce sont des pommades préparées en conditions non stériles (donc non applicables à l'ophtalmologie) dont l'excipient est aqueux. Il n'est pas certain que de telles préparations puissent s'étaler sur l'oeil même en présence d'électrolytes car leur but est de retarder la rupture du gel engendrant ainsi un trouble de la vision. Ils ne peuvent donc trouver d'emploi que pour le traitement des infections des paupières ou du bord externe de l'oeil.

La demande de brevet européen 312.208 montre qu'il est possible de réaliser des gels très visqueux (10.000 à 100.000 cps à température ordinaire) destinés, entre autres, à assurer la cicatrisation des plaies de cornée, c'est-à-dire à présenter une viscosité comparable au fluide baignant le globe oculaire. Dans ce cas, le gel visqueux constitue simplement un fluide lubrifiant assurant un contact aisé et prolongé du facteur de croissance (EGF) avec la partie lésée en cause. Le gel peut être d'une origine quelconque (polyacrylamide, ac.polyacrylique, Pluronic, dérivés de la cellulose).

Si la solution visqueuse est destinée à être employée comme collyre (eye drops), il est préférable que la solution possède une viscosité faible comprise entre 1 et 100 cps. A ce moment la viscosité de la dispersion est trop faible et le liquide s'écoule spontanément de l'oeil.

La référence WO 90/13284 décrit des compositions ophtalmiques contenant un carbomère, un agent anti-allergique : le cromoglycate de sodium ; ces compositions, qui sont normalement acides, sont ajustées à un pH compris entre 5 et 6,8 par adjonction d'une base minérale et en particulier l'hydroxyde de sodium. Ceci nécessite la manipulation d'une quantité relativement importante d'hydroxyde de sodium, ce qui n'est pas sans inconvénients.

L'objet de la présente invention est de remédier aux inconvénients décrits ci-dessus, en réalisant des préparations instillables, stériles: utilisables comme les collyres, c'est-à-dire que l'on peut administrer sous forme de gouttes dans l'oeil, et assurant une adhérence et une rémanence du principe actif prolongée à la surface oculaire par la création par un polymère d'une matrice. Cette bioadhésion résulte de l'interpénétration des chaînes du polymère et d'interactions chimiques de ses groupements fonctionnels carboxyliques avec la couche mucinique du film lacrymal.

La présente invention se rapporte donc à de nouvelles préparations ophtalmiques instillables se présentant sous la forme d'un gel fluide et comprenant une solution aqueuse d'un polymère d'acide acrylique, une solution d'un agent basique soluble dans l'eau, et un agent médicamenteux destiné à l'usage ophtalmique, caractérisées en ce qu'elles présentent un pH compris entre 7,3 et 7,7 et en ce qu'elles sont additionnées d'un agent chélatant choisi dans le groupe constitué par l'acide éthylène diamino tétra acétique, l'acide nitrilo-triacétique et l'acide éthylène diamino NN'-diacétique NN'-dipropionique.

L'agent médicamenteux ou principe actif est choisi dans le groupe constitué par les agents β-bloqueurs (agents anti-glaucomateux), les anti-hypertenseurs oculaires, les agents anti-allergiques, les cicatrisants, les agents antibactériens, les agents antifongiques et les agents antiviraux.

Le but à atteindre, notamment avec les β-bloqueurs ou les agents anti-allergiques, est d'améliorer leur biodisponibilité, sous la forme d'un soluté aqueux possédant une fluidité suffisante et manifeste.

En particulier, la présente invention permet, dans le cas de préparations à base de β-bloquants comme par exemple le maléate de timolol, se présentant ainsi sous forme de gel fluide et correspondant à des concentrations faibles en carbomère (ou carbopol®), d'obtenir une activité aussi importante à la concentration de 0,1 % de timolol que celle obtenue avec du collyre commercial à 0,25 %, tout en manifestant une action plus importante et plus durable à concentration égale On observe ainsi que les compositions à base de maléate de timolol, selon l'invention, manifestent une efficacité plus nette pour une concentration faible, et permettent ainsi de minimiser le passage systémique et par voie de conséquence, les effets secondaires indésirables.

Pour les agents anti-bactériens et/ou anti-viraux, le rôle du polymère d'acide acrylique sera d'assurer la rémanence du principe actif et aussi un meilleur contact avec la muqueuse oculaire.

L'invention est également caractérisée par le fait que le polymère gélifiant est un polymère d'acide acrylique ou carbomère, notamment ceux commercialisés sous les dénominations Carbopol® 934, Carbopol® 940 et Carbopol® 941.

Le polymère d'acide acrylique est ajouté au milieu en quantités faibles s'échelonnant entre 0,05 et 5 % en poids et de préférence entre 0,1 et 1 % de façon à ce qu'après neutralisation par un agent basique, ses groupements fonctionnels soient libérés.

L'agent basique qui sert à ajuster le pH et par voie de conséquence à créer la viscosité de la solution initiale est une base minérale comme un carbonate de métal alcalin ou un bicarbonate de métal alcalin ou bien encore un hydroxyde de métal alcalin. L'agent basique peut être également une base organique comme une dialcoylamine comme la diethylamine, une trialcoylamine comme la triethylamine ou une hydroxyalcoylamine comme l'éthanolamine, la triethanolamine, la N-méthyglucamine ou le trométhanol.

D'une manière préférée, l'agent basique est l'hydroxyde de sodium ou l'hydroxyde de lithium ajouté à doses calculées pour que le pH final de la solution se trouve dans la zone désirée.

Les préparations ophtalmiques selon l'invention contiennent, en outre, un agent conservateur comme par exemple un acide phénol ou un de ses esters, comme les parabens ou les gallates.

L'agent conservateur peut être également un sel d'ammonium quaternaire comme le bromure de Cetrimide, le chlorure de Benzalkonium ou bien le bromure de benzodedecinium.

L'agent complexant ajouté à la préparation, permet notamment d'assurer une meilleure stabilité de la molécule lorsque le principe actif est sensible à la présence de traces métalliques susceptibles de favoriser sa dégradation, comme c'est le cas pour un phénol tel que l'Adrénaline, l'Isoprénaline ou la Dipivefrine; un amino-alcool ou certains antibiotiques du type polypeptidique.

L'agent chélatant est celui défini comme précédemment.

Le rôle de l'agent chélatant sera également de complexer les ions divalents (Calcium, Magnésium) qui ont une influence défavorable sur l'évolution de la forme pendant la durée du stockage.

Les préparations ophtalmiques selon l'invention contiennent également un agent isotonisant, de préférence une molécule organique apte à ajuster la concentration molaire au voisinage de l'isotonie.

Les agents isotonisants les plus appropriés sont les polyols comme par exemple le mannitol, le sorbitol, le xylitol, le dulcitol, le lactitol ou le charmitol qui permettent grâce à leur poids moléculaire élevé, d'ajuster très précisément la quantité d'agent isotonisant requise.

Le principe actif incorporé dans les préparations instillables ophtalmiques, selon l'invention, est en premier lieu un agent β-bloqueur apte à diminuer la pression intraoculaire comme par exemple le propanolol, le pindolol, l'aténolol, le métipranolol, le béfunolol, le cartéolol, le penbutolol, le timolol, le tertatolol et les analogues de structure. Ceux-ci sont utilisés soit sous forme de base, soit plus commodément sous forme de sel d'addition avec un acide minéral ou organique thérapeutiquement acceptable. Leur concentration varie dans de larges proportions selon le niveau d'activité de l'agent β-bloqueur. Elle varie de 0,05 à 5 % selon le produit mais de préférence de 0,05 à 2 %.

Le principe actif est également un agent antibactérien comme un antibiotique choisi dans le groupe constitué par le chloramphénicol, le thiamphénicol, la polymixine, la tétracycline, la thyrocidine, la gramicidine, le colistine méthane sulfonate de sodium, la pénicilline, la néomycine, la gentamicine, la dihydrostreptomycine, l'amikacine, la phosphomycine, la rifamycine ou le sulbactame ; l'agent antibactérien peut être aussi bien un agent synthétique du type quinolone comme la norfloxacine, la pefloxacine, l'ofloxacine, l'amifloxacine, la ciprofloxacine ou la sparfloxacine.

Le principe actif est aussi un agent anti-allergique comme le Nedocromil ou l'acide N-aspartyl glutamique

Le principe actif est également un agent anti-inflammatoire notamment un de ceux susceptibles de réduire la synthèse des prostaglandines par inhibition des prostaglandines-synthétases et/ou des cyclo-oxygénases.

L'agent antiviral utilisé comme principe actif dans les compositions selon l'invention est un dérivé de base purique comme le fluorouracile, la trifluorothymidine, la brivudine, l'idoxuridine, la vidarabine, l'acycloguanosine, la fiacitabine, la cytarabine, la zidovudine, l'acyclovir. Ces composés sont utilisés sous forme de base ou plus commodément de sels d'addition avec un acide minéral ou organique. La concentration en agent antiviral s'échelonne entre 0,01 et 2 % selon le produit.

L'invention concerne également un procédé d'obtention des préparations ophtalmiques instillables selon l'invention, caractérisé en ce que l'on dissout ou disperse le polymère d'acide acrylique dans un milieu aqueux, on ajoute une autre solution préparée distinctement contenant un agent conservateur, un agent chélatant et un agent d'isotonie, on mélange les deux solutions, on ajoute l'agent médicamenteux avant ou après l'agent basique et on mélange jusqu'à obtention d'un mélange homogène dont le pH est compris entre 7,3 et 7,7.

On peut également incorporer directement le principe actif à une des solutions aqueuses. Les solutions ou dispersions ou le principe actif lui-même sont stérilisés par les moyens physiques appropriés. Le mélange est effectué ensuite.

Les compositions ophtalmiques instillables selon l'invention trouvent un emploi dans le traitement des affections oculaires notamment pour traiter le glaucome à angle ouvert (GAO), le glaucome par fermeture de l'angle, les infections oculaires d'origine microbiennes ou virales, les phénomènes d'allergie, les inflammations, le traitement des kératites. La posologie va de 1 à 5 gouttes dans chaque oeil, par jour, répartie en autant d'instillations.

La présence du polymère d'acide acrylique conduit à une réduction notable du nombre d'instillations.

Les exemples suivants illustrent l'invention :

## EXEMPLE I

## COMPOSITION OPHTALMIQUE INSTILLABLE A BASE DE TIMOLOL

| Composition : | |
|---|---|
| • Polymère acrylique commercialisé sous la dénomination Carbopol® 940 | 10 g |
| • Ethylène diamino-tétraacétate de sodium | 0,50 g |
| • Bromure de cétrimide | 0,50 g |
| • Sorbitol | 200 g |
| • Timolol (sous forme de Maléate) | 6,83 g |
| • Na OH | 4,40 g |
| • Eau | 4778 g |
| Pour un total de | 5000 g environ |

Dans un ballon de 5 litres, on introduit 2 litres d'eau distillée puis sous agitation par petites fractions, 10 g de polymère acrylique à environ 500 t/mn. On agite jusqu'à obtention d'une solution homogène sans grumeaux.

Dans un autre volume d'eau, on dissout le bromure de Cétrimide, l'acide éthylène diamino tétraacétique (sel de sodium) et le sorbitol. Lorsque la solution est complète, on l'ajoute à la solution de polymère acrylique préparée précédemment. Le mélange est stérilisé en cuve, à la chaleur (121° pendant 20 mn).

Dans un bécher renfermant environ 200 ml d'eau, on dissout 6,83 g de Maléate de Timolol que l'on stérilise par filtration stérilisante sous pression et fait passer dans la cuve où se trouve le mélange stérile.

On prépare, en outre, une solution de 4,5 g d'hydroxyde de sodium dans 45 ml d'eau purifiée. On dissout sous agitation.

A chaque opération, on rince les récipients avec un petit volume d'eau et ajuste si nécessaire, au poids d'eau prescrit.

Ensuite et dans les mêmes conditions, on fait passer la solution stérile d'hydroxyde de sodium dans la cuve de fabrication, on homogénéise pendant une heure. On vérifie sur un prélèvement la valeur du pH et la viscosité.

On obtient ainsi une solution visqueuse de Timolol renfermant 0,1 % de principe actif.

## EXEMPLE II

## ETUDE DE L'ACTION ANTI-HYPERTENSIVE DE LA COMPOSITION DE L'EXEMPLE I

On détermine une hypertension intra-oculaire expérimentale chez le lapin puis on administre dans un oeil une goutte d'une préparation selon l'invention renfermant, à titre de principe actif, 0,10 % de Timolol sous forme de maléate et des concentrations croissantes de polymère d'acide acrylique. On mesure après 15 mn de latence, les variations de la pression intra-oculaire chez chaque animal en comparaison avec celle d'un lot de lapins hypertendus, non traités, et celles occasionnées par un collyre à 0,1 % du commerce.

Ces mesures sont répétées pendant 8 à 10 heures et permettent de déterminer les pourcentages de variation de la pression intra-oculaire pendant cette période.

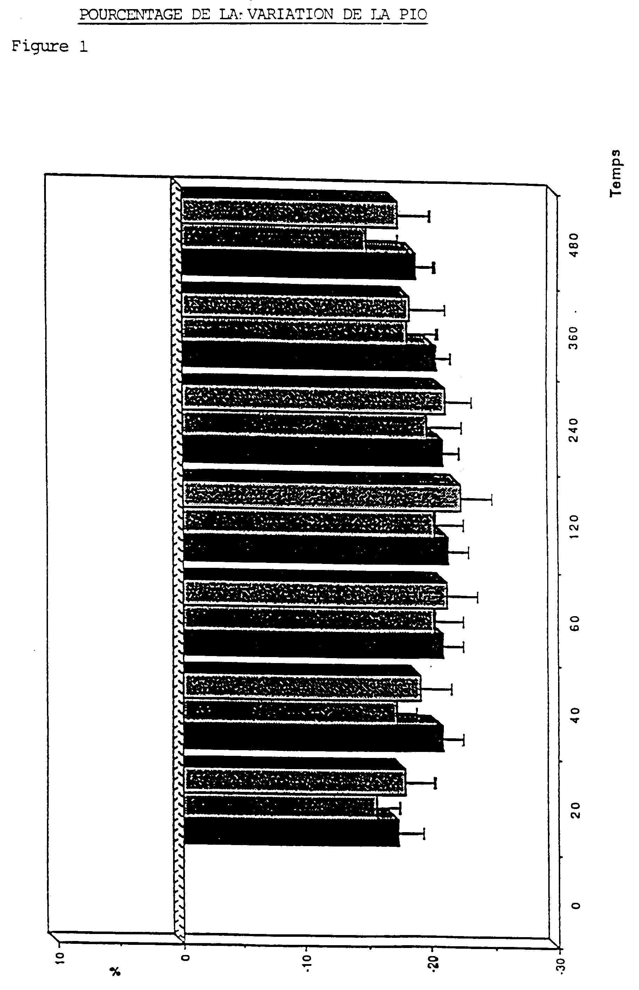

On constate que, d'une manière constante, les compositions selon l'invention renfermant seulement 0,1 % de polymère d'acide acrylique et 0,1 % de Timolol fournissent la réduction la plus importante de la pression intra-oculaire pendant 8 heures d'observation. Les solutions contenant 0,2 et 0,35 % de polymère d'acide acrylique donnent des résultats peu différents entre elles et souvent très proches de ceux obtenus avec la solution à 0,1 % de polymère acrylique (Figure 1).

Dans une deuxième expérience, on a comparé les effets sur la pression intra-oculaire du lapin hypertendu, d'une solution de maléate de Timolol à 0,1 % du commerce et une composition ophtalmique selon l'invention renfermant 0,1 % de Timolol sous forme de Maléate.

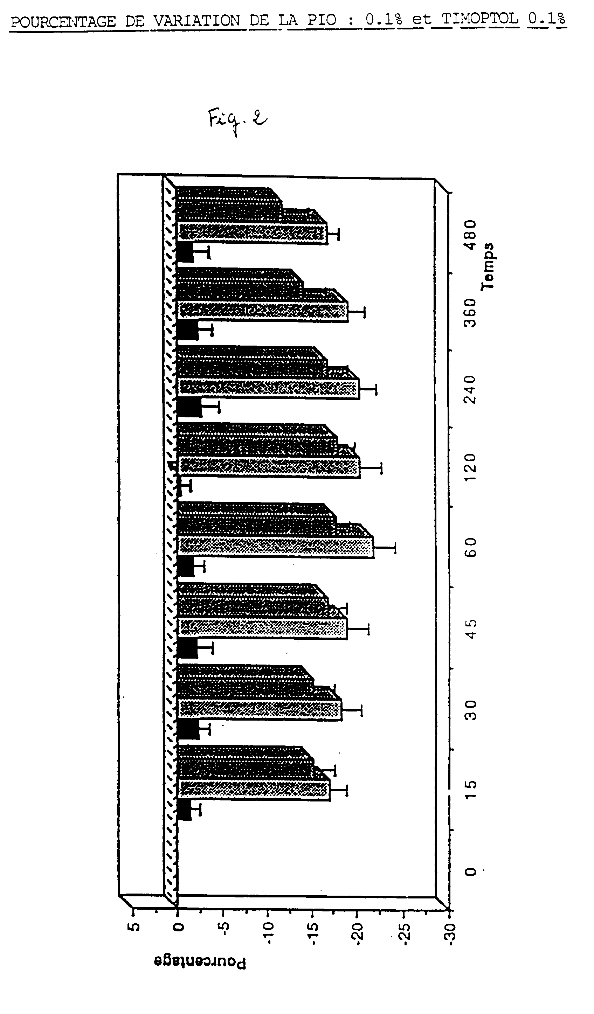

Les résultats obtenus sont rassemblés dans la Figure 2.

4

Ils montrent que sur une période de mesure de 8 heures, la pression oculaire baisse sensiblement plus, à concentration en principe actif égale avec les compositions selon l'invention qu'avec les solutions habituelles du même principe actif.

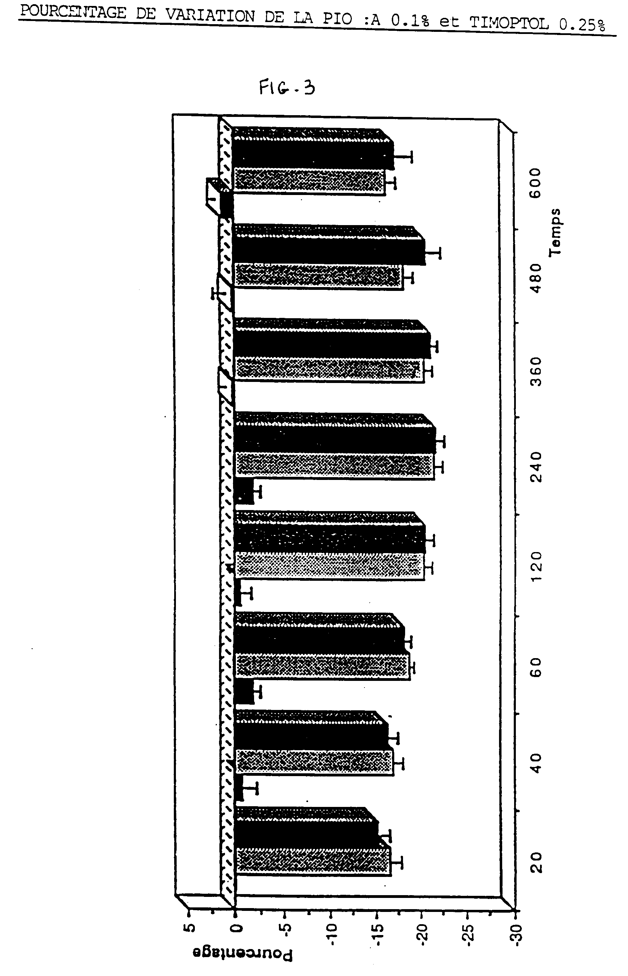

Dans une troisième série d'expériences effectuées avec une solution commerciale de Timolol, sous forme de maléate à 0,25 % de principe actif et avec une composition selon l'invention renfermant le Timolol à la dose de 0,1 % sous forme de. maléate, on a obtenu une diminution de la pression intra-oculaire du lapin pratiquement aussi prolongée et aussi importante avec la solution selon l'invention, sensiblement plus diluée, qu'avec la préparation commerciale (Figure 3) sans qu'il y ait de différence statistiquement significative.

## EXEMPLE III

## PREPARATION INSTILLABLE A BASE DE TROXERUTINE

| | |
|---|---|
| • Polymère d'acide acrylique commercialisé sous la dénomination Carbopol® 940 | 7,50 g |
| • Ethylène diamino-tétracétate de sodium | 0,50 g |
| • Bromure de cétrimide | 0,50 g |
| • Sorbitol | 200 g |
| • Troxérutine | 5,00 g |
| • Hydroxyde de sodium | 4,00 g |
| • Eau | 4783 g |

## MODE OPERATOIRE

a) Préparation de la solution de polymère d'acide acrylique

Dans un ballon de 5 litres, on introduit 750 ml d'eau et sous agitation de 500 t/mn environ, on ajoute peu à peu 7,50 g de polymère d'acide acrylique et on poursuit l'agitation jusqu'à obtention d'une solution homogène sans grumeaux.

b) Solution de tétracémate de sodium

Dans 1000 ml d'eau, on ajoute successivement et sous agitation le tétracémate de sodium (0,50 g) puis le bromure de cétrimide (0,50 g) et enfin, le sorbitol (200 g).
On transvase dans le ballon de 5 litres de cette solution, on rince le récipient avec 250 ml d'eau et on homogénéise.
La solution totale est transférée dans une cuve. On rince à nouveau le ballon avec 250 ml d'eau et on stérilise le mélange à la chaleur.

c) Préparation de la solution de neutralisation.

Dans 40 ml d'eau, on dissout sous agitation 4,00 g d'hydroxyde de sodium. On met 33 ml de la solution ainsi réalisée dans la chambre de pression pour filtration ultérieure.
Dans un bécher contenant 1 993 ml d'eau, on introduit 7 ml restant de la solution de soude préparée immédiatement auparavant sous agitation. On homogénéise la solution dont on vérifie le pH (pH = 12,2) et que l'on fait passer progressivement dans un bécher contenant 5,00 g de Troxerutine exactement pesés. On maintient l'agitation presqu'à achèvement de l'addition. La solution de Troxerutine ainsi réalisée a un pH de 10,0.
Dans la cuve, en procédant sous agitation, on filtre stérilement les 33 ml de solution de soude pour neutralisation sur filtre stérilisant et sous pression en conditions stériles.
On rince par ailleurs le bécher et le filtre, avec 250 ml d'eau.
Dans les mêmes conditions, on filtre sur filtre stérilisant dans des conditions de stérilité, la solution de troxerutine et on rince le récipient. On la fait passer dans la cuve contenant déjà le polymère d'acide acrylique, on homogénéise pendant une heure puis on prélève stérilement un échantillon pour vérifier le pH (7,4 à 7,7). On laisse reposer 24 heures puis on homogénéise encore 10 mn puis on prélève à nouveau un échantillon dont on vérifie le pH et la viscosité. La solution visqueuse est prête pour le conditionnement en flacons de 5 ml.

## EXEMPLE IV

### PREPARATION A BASE DE 17$\alpha$-ACETOXY 3,20-DIOXO 6-METHYL 19-NOR PREGNA 4,6-DIENE

On prépare, comme aux exemples précédents, une solution homogène de 7,50 g de polymère acrylique dans 2 000 ml d'eau.

Dans un ballon renfermant 1 731 ml d'eau distillée, on ajoute successivement et sous agitation :

| | |
|---|---|
| • Tétracémate de sodium | 0,50 g |
| • Bromure de cétrimide | 0,50 g |
| • Sorbitol | 200,00 g |

Cette solution est versée dans le récipient contenant déjà la solution de polymère d'acide acrylique. On rince le ballon avec 250 ml d'eau et on homogénéise la solution totale. Cette solution est ensuite transvasée dans la cuve de mélange et on y ajoute à nouveau 250 ml d'eau provenant du rinçage du ballon. On stérilise le mélange à la chaleur.

On prépare ensuite une solution de 3,20 g d'hydroxyde de sodium dans 32 ml d'eau puis on transvase la solution d'hydroxyde de sodium sur un filtre stérilisant sous pression en conditions stériles.

On rince ensuite le bécher et le filtre stérilisant avec 250 ml d'eau. On ouvre alors la cuve et sous agitation on saupoudre en atmosphère stérile, le 17$\alpha$-acétoxy 3,20-dioxo 6-méthyl 19-nor pregna 4,6-diene puis on rince le récipient ayant contenu la poudre avec 250 ml d'eau stérile.

On referme la cuve et on procède à l'homogénéisation pendant 1 heure puis on prélève stérilement un échantillon ainsi réalisé pour vérifier le pH (pH 7,3 à 7,7) et le titre en 17$\alpha$-acétoxy 3,20-dioxo 6-méthyl 19-nor pregna 4,6-diène (98 à 102 % de la théorie).

Après 24 heures de repos, on homogénéise pendant 10 mn puis on prélève à nouveau un échantillon. Après avoir vérifié le pH et la viscosité, on répartit en conditionnements individuels de 5 g.

## EXEMPLE V

### COMPOSITION OPHTALMIQUE INSTILLABLE A BASE DE MICRONOMICINE

| Composition : | |
|---|---|
| • Polymère acrylique (Carbopol® 940) | 6,00 g |
| • Carboxyméthyl cellulose | 25,00 g |
| • EDTA | 0,50 g |
| • Mannitol | 200 g |
| • Benzalkonium chlorure | 0,50 g |
| • Micronomicine | 15,00 g |
| • Na OH 10 % | qs pH = 7,4 |
| • Eau purifiée ou distillée | qs 5000 g |

Dans un ballon de 5 litres, on introduit 2 litres d'eau purifiée, puis le carbopol® 940 par petites fractions sous agitation à environ 500 t/mn. On poursuit l'agitation jusqu'à l'obtention d'une solution homogène sans grumeaux.

Dans un autre volume d'eau, on dissout le chlorure de benzalkonium, l'EDTA et le mannitol. Lorsque la dissolution est complète, on l'ajoute à la solution de polymère acrylique préparée précédemment. Le mélange est stérilisé en cuve, à la chaleur (121° pendant 20 mn).

Dans un bécher renfermant 1 000 ml d'eau, on dissout 15 g de micronomicine et 5 g de carboxy méthyl cellulose. L'ensemble est stérilisé par filtration stérilisante sous pression et passé dans la cuve où se trouve le mélange stérile.

On fait passer la solution stérile de soude obtenue dans les mêmes conditions dans la cuve de fabrication. On ajuste à pH = 7,4, on homogénéise pendant une heure. On complète à 5 000 g avec de l'eau purifiée stérile et on homogénéise une heure.

On obtient une solution visqueuse de micronomicine renfermant 0,3 % de principe actif.

## EXEMPLE VI

## COMPOSITION OPHTALMIQUE INSTILLABLE A BASE DE VITAMINE B.12

| Composition : | |
|---|---|
| • Carbopol® 980 | 10,00 g |
| • EDTA | 0,50 g |
| • Sorbitol | 200 g |
| • Bromure de cétrimide | 0,50 g |
| • Vitamine B.12 | 2,50 g |
| • Na OH 10 % | qs pH = 7,4 |
| • Eau purifiée | qs 5000 g |

Dans un ballon de 5 litres, on introduit 4,5 litres d'eau purifiée puis l'EDTA, le sorbitol, le bromure de cétrimide et la vitamine B.12. Après dissolution, on ajoute, par petites fractions, le carbopol® 980 sous agitation jusqu'à l'obtention d'un gel homogène.

Le mélange est stérilisé en cuve, à la chaleur de 121° pendant 20 mn.

On ajoute une solution de soude, stérilisée par filtration stérilisante jusqu'à pH = 7,4. On homogénéise une heure.

On complète à 5 000 g avec de l'eau purifiée stérile et on homogénéise une heure.

On obtient une solution visqueuse de vitamine B.12 renfermant 0,05 % de principe actif.

## EXEMPLE VII

## COMPOSITION OPHTALMIQUE INSTILLABLE A BASE DE NANDROLONE

| Composition : | |
|---|---|
| • Carbopol® 934P | 7,50 g |
| • Chitosan | 16,00 g |
| • EDTA | 0,50 g |
| • Sorbitol | 200 g |
| • Mercurothiolate de sodium | 0,10 g |
| • Mandrolone | 50,00 g |
| • Na OH | qs pH = 7,4 |
| • Eau purifiée | qs 5000 g |

Dans un ballon de 5 litres, on introduit 2 litres d'eau purifiée, puis sous agitation, le carbopol® 934P par petites fractions à environ 500 t/mn. On agite jusqu'à l'obtention d'une solution homogène sans grumeaux.

Dans un autre volume d'eau, on dissout l'EDTA, le sorbitol et le mercurothiolate de sodium. Après dissolution totale, on ajoute la solution obtenue à celle de polymère acrylique. Le mélange est stérilisé en cuve, à la chaleur, à 121° pendant 20 mn.

Dans un bécher renfermant 1 000 ml d'eau, on dissout la nandrolone et le chitosan. L'ensemble est stérilisé par filtration stérilisante et ajouté au mélange présent dans la cuve.

On fait passer la solution stérile de soude obtenue dans la cuve de fabrication jusqu'à pH = 7,4. On homogénéise pendant une heure.

On complète à 5 000 g avec de l'eau purifiée stérile.

On obtient une solution visqueuse de nandrolone renfermant 1 % de principe actif.

## EXEMPLE VIII

**COMPOSITION OPHTALMIQUE INSTILLABLE A BASE DE NEDOCROMIL**

| Composition : | |
| --- | --- |
| • Carbopol® 941 | 5,00 g |
| • Dextran | 25,00 g |
| • Polyéthylène glycol 4000 | 25,00 g |
| • EDTA | 0,50 g |
| • Mannitol | 185 g |
| • Benzalkonium chlorure | 0,50 g |
| • Nedocromil sel disodique | 100,00 g |
| • Na OH | qs pH = 7,4 |
| • Eau purifiée | qs 5000 g |

Dans un ballon de 5 litres, on introduit 1,5 litres d'eau purifiée, puis, sous agitation, le carbopol 941 par petites fractions à environ 500 t/mn. On agite jusqu'à l'obtention d'une solution homogène sans grumeaux.

Dans un autre volume d'eau, on dissout le dextran, le polyéthylèneglycol, l'EDTA, le mannitol et le chlorure de benzalkonium. Après dissolution totale, on ajoute la solution obtenue à celle de polymère acrylique. Le mélange est stérilisé en cuve, à la chaleur, à 121° pendant 20 mn.

Dans un bécher renfermant 2 litres d'eau, on dissout le nedrocromil sel disodique. L'ensemble est stérilisé par filtration stérilisante et ajouté au mélange présent dans la cuve.

On fait passer la solution stérile de soude obtenue dans la cuve de fabrication jusqu'à pH = 7,2. On homogénéise pendant une heure.

On complète à 5 000 g avec de l'eau purifiée stérile.

On obtient une solution visqueuse de nedrocromil sel disodique renfermant 2 % de principe actif.

En opérant de la même façon, on peut obtenir une composition ophtalmique instillable à base d'acide N-acétyl aspartyl glutamique.

## Revendications

1. Préparation ophtalmique instillable se présentant sous la forme d'un gel fluide et comprenant une solution aqueuse d'un polymère d'acide acrylique, une solution d'un agent basique soluble dans l'eau, et un agent médicamenteux destiné à l'usage ophtalmique, caractérisée en ce qu'elle présente un pH compris entre 7,3 et 7,7 et en ce qu'elle est additionnée d'un agent chélatant choisi dans le groupe constitué par l'acide éthylène diamino tétra acétique, l'acide nitrilo-triacétique et l'acide éthylène diamino NN'-diacétique NN'-dipropionique.

2. Préparation ophtalmique selon la revendication 1, caractérisée en ce que l'agent chélatant est l'acide éthylène diamino tétra acétique.

3. Préparation ophtalmique selon la revendication 1, caractérisée en ce que l'agent chélatant est l'acide nitrilotriacétique.

4. Préparation ophtalmique selon la revendication 1, caractérisée en ce que l'agent chélatant est l'acide éthylène diamino NN'-diacétique NN'-dipropionique.

5. Préparation ophtalmique selon l'une des revendications 1 à 4, caractérisée en ce que la concentration en agent chélatant est d'environ 0,1 g par litre.

6. Préparation ophtalmique selon l'une des revendications 1 à 5, caractérisée en ce que l' agent médicamenteux est choisi dans le groupe constitué par les agents β-bloqueurs, les antihypertenseurs oculaires, les agents cicatrisants, les agents anti-allergiques, les agents antibactériens, les agents anti-viraux et les agents anti-fongiques.

7. Préparation ophtalmique selon l'une des revendications 1 à 6, caractérisée en ce que l'agent β-bloqueur est choisi dans le groupe constitué par le propanolol, le carteolol, le pindolol, l'atenolol, le metipranolol, le befunolol, le penbutolol, le timolol, le tertatolol, sous forme de base ou de sels d'addition avec un acide minéral ou organique.

8. Préparation ophtalmique selon l'une des revendications 1 à 6, caractérisée en ce que l'agent antibactérien est un antibiotique.

9. Préparation ophtalmique selon l'une des revendications 1 à 6, caractérisée en ce que l'agent antibactérien est un agent synthétique du type quinolone.

10. Préparation ophtalmique selon l'une des revendications 1 à 6, caractérisée en ce que l'agent anti-viral est choisi dans le groupe constitué par la fluorouracile, l'idoxuridine, la zidovudine, la cytarabine et la cytarabine phosphorylée.

11. Un procédé d'obtention des préparations ophtalmiques selon l'une des revendications 1 à 10, caractérisé en ce que l' on disperse le polymère d'acide acrylique dans un milieu aqueux, on ajoute une autre solution préparée distinctement contenant un agent conservateur, un agent chélatant et un agent d'isotonie, on mélange les deux solutions, on ajoute l'agent médicamenteux avant ou après l'agent basique et on mélange jusqu'à obtention d'un mélange homogène dont le pH est compris entre 7,3 et 7,7.

## Claims

1. An instillable ophthalmic preparation in the form of a fluid gel and which comprises an aqueous solution of an acrylic acid polymer, a solution of a water-soluble basic agent, and a medicinal agent intended for ophthalmic use, characterised in that it has a pH between 7.3 and 7.7 and in that to which is added a chelating agent selected from the group consisting of ethylenediaminetetraacetic acid, nitrilotriacetic acid and ethylenediamine-N,N'-diacetic-N,N'-dipropionic acid.

2. The ophthalmic preparation according to claim 1, characterised in that the chelating agent is ethylenediaminetetraacetic acid.

3. The ophthalmic preparation according to claim 1, characterised in that the chelating agent is nitrilotriacetic acid.

4. The ophthalmic preparation according to claim 1, characterised in that the chelating agent is ethylenediamine-N,N'-diacetic-N,N'-dipropionic acid.

5. The ophthalmic preparation according to one of claims 1 to 4, characterised in that the concentration of chelating agent is about 0.1 g per litre.

6. The ophthalmic preparation according to one of claims 1 to 5, characterised in that the medicinal agent is selected from the group consisting of β-blockers, ocular antihypertensive agents, healing agents, anti-allergy agents, anti-bacterial agents, anti-viral agents, and anti-fungal agents.

7. The ophthalmic preparation according to one of claims 1 to 6, characterised in that the β-blocker is selected from the group consisting of propanolol, carteolol, pindolol, atenolol, metipranolol, befunolol, penbutolol, timolol, tertatolol, in the form of a base or an addition salt with a mineral or organic acid.

8. The ophthalmic preparation according to one of claims 1 to 6, characterised in that the anti-bacterial agent is an antibiotic.

9. The ophthalmic preparation according to one of claims 1 to 6, characterised in that the anti-bacterial agent is a synthetic agent of the quinolone type.

10. The ophthalmic preparation according to one of claims 1 to 6, characterised in that the anti-viral agent is selected from the group consisting of fluorouracil, idoxuridine, zidovudine, cytarabine, and phosphorylated cytarabine.

**11.** A method of obtaining ophthalmic preparations according to one of claims 1 to 10, characterised in that the acrylic acid polymer is dispersed in an aqueous medium, another solution containing a preservative, a chelating agent and an isotonia agent, prepared separately, is added, the two solutions are mixed, the medicinal agent is added before or after the basic agent, and mixing is carried out until a homogeneous mixture is obtained the pH of which is between 7.3 and 7.7.

**Patentansprüche**

**1.** Instillierbare ophthalmische Zubereitung, welche in Form eines Fluidgels vorliegt und eine wässerig Lösung eines Acrylsäure-Polymers, eine Lösung eines wasserlöslichen basischen Mittels und eines Arzneimittels zur ophthalmischen Verwendung umfaßt, dadurch gekennzeichnet, daß sie einen pH zwischen 7,3 und 7,7 aufweist, und daß ihr ein Chelatbildner, ausgewählt aus der Gruppe bestehend aus Ethylendiamintetraessigsäure, Nitrilotriessigsäure und Ethylendiamin-N,N'-diessig-N,N'-dipropionsäure, zugesetzt wurde.

**2.** Ophthalmische Zubereitung nach Anspruch 1, dadurch gekennzeichnet, daß der Chelatbildner Ethylendiamintetraessigsäure ist.

**3.** Ophthalmische Zubereitung nach Anspruch 1, dadurch gekennzeichnet, daß der Chelatbildner Nitrilotriessigsäure ist.

**4.** ophthalmische Zubereitung nach Anspruch 1, dadurch gekennzeichnet, daß der Chelatbildner Ethylendiamin-N,N'-diessig-N,N'-dipropionsäure ist.

**5.** Ophthalmische Zubereitung nach einem der Ansprüche 1 bis 4, dadurch gekennzeichnet, daß die Konzentration des Chelatbildners etwa 0,1 g pro Liter beträgt.

**6.** Ophthalmische Zubereitung nach einem der Ansprüche 1 bis 5, dadurch gekennzeichnet, daß das Arzneimittel ausgewählt ist aus der Gruppe bestehend aus β-Blockern, okularen Antihypertensiva, Vernarbungsmitteln, Antiallergika, antibakteriellen Mitteln, antiviralen Mitteln und Fungiziden.

**7.** Ophthalmische Zubereitung nach einem der Ansprüche 1 bis 6, dadurch gekennzeichnet, daß der β-Blocker ausgewählt ist aus der Gruppe bestehend aus Propanolol, Carteolol, Pindolol, Atenolol, Metipranolol, Befunolol, Penbutolol, Timolol, Tertatolol, in Form einer Base oder eines Säureadditionssalzes mit einer Mineral- oder organischen Säure.

**8.** Ophthalmische Zubereitung nach einem der Ansprüche 1 bis 6, dadurch gekennzeichnet, daß das antibakterielle Mittel ein Antibiotikum ist.

**9.** Ophthalmische Zubereitung nach einem der Ansprüche 1 bis 6, dadurch gekennzeichnet, daß das antibakterielle Mittel ein synthetisches Mittel vom Chinolon-Typ ist.

**10.** Ophthalmische Zubereitung nach einem der Ansprüche 1 bis 6, dadurch gekennzeichnet, daß das antivirale Mittel ausgewählt ist aus der Gruppe bestehend aus Fluorouracil, Idoxuridin, Zidovudin, Cytarabin und phosphoryliertem Cytarabin.

**11.** Verfahren zum Erhalten ophthalmischer Zubereitungen nach einem der Ansprüche 1 bis 10, dadurch gekennzeichnet, daß das Acrylsäure-Polymer in einem wässerigen Medium dispergiert wird, eine weitere, getrennt hergestellte Lösung, die ein Konservierungsmittel, einen Chelatbildner und ein Isotoniemittel enthält, zugesetzt wird, die beiden Lösungen gemischt werden, das Arzneimittel vor oder nach dem basischen Mittel zugesetzt wird, und gemischt wird, bis eine homogene Mischung erhalten wird, deren pH zwischen 7,3 und 7,7 liegt.

POURCENTAGE DE LA VARIATION DE LA PIO

Figure 1

POURCENTAGE DE VARIATION DE LA PIO : 0.1% et TIMOPTOL 0.1%

Fig. 2

POURCENTAGE DE VARIATION DE LA PIO :A 0.1% et TIMOPTOL 0.25%

FIG.3